# EUROPEAN PATENT APPLICATION

(11) **EP 3 695 842 A1**
(43) Date of publication of application: **19.08.2020**
(21) Application number: 18866610.1
(22) Date of filing: 11.10.2018
(51) Int. Cl.: A61K 31/635, A61P 43/00

(54) **USE OF SULFASALAZINE AS AN INHIBITOR OF THE FORMATION OF ADVANCED GLYCATION END PRODUCTS**

(30) Priority: 12.10.2017 RU 2017136164
(71) Applicant: The Limited Company Research And Development Company "Medbiopharm", Obninsk, Kaluga Region 249030 (RU)
(72) Inventor: SHTYRLIN, Yurij Grigorevich, Kazan Res. Tatarstan 420133 (RU); SPASOV, Aleksandr Alekseevich, Volgograd Volgogradskoy Obl 400066 (RU); BALAKIN, Konstantin Valerevich, Shchelkovo Moskovskoy Obl. 141100 (RU); KUZNETSOVA, Valentina Andreevna, Volgograd Volgogradskoy Obl. 400048 (RU); PETROV, Vladimir Ivanovich, Volgograd Volgogradskoy Obl. 400066 (RU); STRELNIK, Aleksej Dmitrievich, Kazan Res. Tatarstan 420124 (RU)
(74) Representative: Vitina, Maruta
(86) International application number: PCT/RU2018/050127
(87) International publication number: WO 2019/074406

(57) **Abstract**

The invention relates to the field of healthcare, namely, to the treatment of socially significant diseases, such as complications of diabetes mellitus, atherosclerosis, rheumatoid arthritis, osteoarthritis, neurodegenerative diseases including Alzheimer's and Parkinson's diseases, cataract, age-related diseases, etc.

The essence of the invention is the use of sulfasalazine (international non-proprietary name; synonyms: Salazosulfapyridine, Azopyrine, Asulfidine, Salazopyridin, Salazopyrin, Salazosulfapyridine, Salicylazosulfapyridin, Salisulf, Sulfasalazyn, Sulphasalazine) of the general formula I as an inhibitor of the formation of advanced glycation end-products:

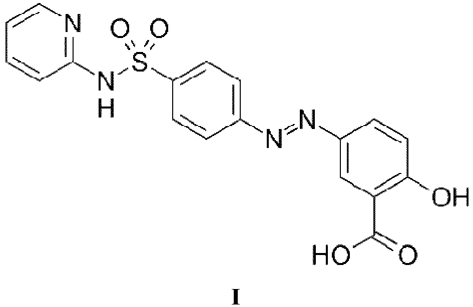

## Description

The invention relates to the use of Sulfasalazine (international non-proprietary name; synonyms: Salazosulfapyridine, Azopyrine, Asulfidine, Salazopyridin, Salazopyrin, Salazosulfapyridine, Salicylazosulfapyridin, Salisulf, Sulfasalazyn, Sulphasalazine) of general formula I for a new purpose as an inhibitor of the formation of advanced glycation end-products (hereinafter, AGEs):

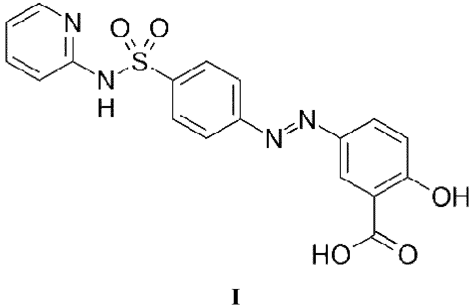

Sulfasalazine I is an effective inhibitor of the formation of glycation end products and can be widely used in medicine in the treatment of socially significant diseases, namely, complications of diabetes mellitus, atherosclerosis, rheumatoid arthritis, osteoarthritis, neurodegenerative diseases, including Alzheimer's and Parkinson's diseases, cataracts, age related diseases, etc.

A prior art study by the applicant revealed that sulfasalazine was introduced to the market in the 1950s as a treatment for rheumatoid arthritis, Crohn's disease and ulcerative colitis ([1] M.A. Peppercorn, Ann. Intern. Med., 1984, 101: 377-386; [2] G.L. Plosker, K.F. Croom, Drugs, 2005, 65: 1825-1849). The mechanism of action of sulfasalazine and its metabolites is associated primarily with the inhibition of the activation of nuclear factor kappa B (NFKB), which is contained in cells of all types and is associated with the development of inflammatory and autoimmune diseases, as well as septic shock. Sulfasalazine can accumulate in the connective tissue of the intestine with the release of 5-aminosalicylic acid, which has an anti-inflammatory effect, and sulfapyridine, which has an antimicrobial effect against *diplococci, streptococci, gonococci,* and *Escherichia coli.*

However, an analysis of literature and patent information sources did not reveal information about the antiglycan activity of sulfasalazine. Thus, the prior art analysis by the applicant did not reveal any data on the possibility of using Sulfasalazine (Formula I) or its saline and co-crystalline forms as inhibitors of the formation of advanced glycation end products.

Glycation (non-enzymatic glycosylation, Maillard reaction) is a chemical reaction in which the carbonyl groups of reduced sugars bind to the amino groups of long-lived proteins, lipids or peptides, with the formation of advanced glycation end-products (AGEs) ([3] S. Khangholi, F.A. Abdul Majid, N.J.A. Berwary, F. Ahmad, R. Bin Abd Aziz, Planta Med., 2016; 82: 32-45; [4] V.P. Singh, A. Bali, N. Singh, A.S. Jaggi, Korean J. Physiol. Pharmacol., 2014, 18: 1-14).

At the same time, intracellular and extracellular accumulation of AGEs is considered an important factor in the pathogenesis of diseases, such as atherosclerosis ([5] M. Busch, S. Franke, C. Rüster, G. Wolf, European Journal of Clinical Investigation, 2010, 40(8): 742-755), heart failure, inflammation, rheumatoid arthritis and osteoarthritis, neurodegenerative diseases, including Alzheimer's and Parkinson's diseases ([6] J. Li, D. Liu, L. Sun, Y. Lu, Z. Zhang, Journal of the Neurological Sciences, 2012, 317: 1-5), other age-related diseases, and cataract ([7] I. Sadowska-Bartosz I,G. Bartosz, Mech. Ageing Dev. 2016, 160: 1-18). This process is very intense in diabetes mellitus, with the rate of AGEs formation depending on the concentration and duration of action of glucose ([8] R. Ramasamy, S.F. Yan, A.M. Schmidt, Ann. N. Y. Acad Sci., 2011, 1243: 88-102; [9] M. I. Balabolkin, Diabetes Mellitus, 2002, 4: 8-16). The effects of AGEs can be classified as receptor-independent or receptor-dependent, and AGEs can act intracellularly or circulate and act on cell surface receptors, such as receptor for advanced glycation end products (RAGE). Since glycation occurs over a long period of time, AGEs affect long-lived proteins. For example, the main targets for them are the structural components of connective tissue and, in particular, type IV collagen, as well as other long-lived proteins, including myelin, tubulin, crystallin, plasminogen activator 1, and fibrinogen, which can also undergo glycation ([10] S.-Y. Goh, M. E. Cooper. J Clin Endocrinol Metab, 2008, 93(4): 1143-1152). By binding to membrane RAGEs, glycation end-products activate certain intracellular signaling pathways leading to increased formation of pro-inflammatory cytokines, free radicals and chemoattractants ([3]; [11] S.C. Ho, P.W. Chang, Am. J. Plant. Sci., 2012, 3: 995-1002). All of the above is the basis of the pathogenesis of such consequences of diabetes mellitus as diabetic atherosclerosis, nephro-, neuro-, retino-, cardio-, and angiopathies, which account for the high risk of disability and mortality among patients with diabetes mellitus.

The use of compounds with high antiglycation activity will reduce the formation of AGEs in the body, thereby improving the quality of life of patients by reducing the risk of atherosclerosis, cataract, rheumatoid arthritis, osteoarthritis, neurodegenerative diseases including Alzheimer's and Parkinson's diseases, as well as such complications of diabetes mellitus as diabetic atherosclerosis, nephro-, neuro-, retino-, cardio-, and angiopathies, which account for the high risk of disability and mortality among patients with diabetes mellitus.

All of the above arouses heightened worldwide interest in the search for inhibitors of advanced glycation end-products, since **there are no drugs that specifically inhibit the formation of AGEs** and **are approved for clinical use in the world as of the date of submission of this application.**

The applicant's analysis of Russian and foreign patent databases, scientific literature and Internet resources showed that there are analogues of the claimed technical solution as to their intended purpose, which can inhibit the formation of AGEs yet **have significant disadvantages, namely insufficient efficacy and/or significant undesirable effects, e.g. are highly toxic, etc.**

Further, the applicant provides information on the identified drugs that have reached the stage of clinical trials. The first and most studied substance of those inhibiting protein glycation is aminoguanidine (AG) ([12] *A. Cerami, P.C. Ulrich, M. Brownlee,* Pat US4758583A, published 19.07.1988). It is designed to prevent the formation of AGEs and glucose-derived cross-linked collagen molecules. The mechanism of the antiglycation action of aminoguanidine is associated with its ability to capture reactive dicarbonyl intermediates. **However, clinical trials of this drug were stopped due to lack of efficacy and the presence of undesirable effects.**

Currently, clinical trials of pyridoxamine are being conducted ([13] *R. Khalifah, B.G. Hudson, Pat* US6716858B1, published 06.04.2004*).* Pyridoxamine also has antiglycation properties, but **it also shows low activity comparable to that of aminoguanidine, which has been withdrawn from clinical trials.** As of the date of submission of this application, the applicant has not discovered any other analogues of the claimed technical solution used for its intended purpose and included in the stage of clinical trials.

Thus, as of the date of submission of the application materials, the problem of creating highly active inhibitors of the formation of AGEs approved for clinical use remains unresolved not only in the Russian Federation, but also abroad.

The claimed technical solution is illustrated by the following materials:
- Table 1 showing the antiglycation activity of sulfasalazine compared with the prototype (aminoguanidine).
- Table 2 showing the values of the 50% inhibitory concentration of sulfasalazine compared with the prototype (aminoguanidine).

The object of the claimed technical solution is the search for new uses of known drugs having, in addition to the known field of application, high antiglycation activity, which ensures the accelerated market launch of the drug with a new therapeutic indication.

The technical result of the proposed invention is the use of sulfasalazine (a well-known medication for the treatment of rheumatoid arthritis, Crohn's disease and ulcerative colitis) as an inhibitor of the formation of AGEs, since it has a higher antiglycation activity compared to substances that have reached the stage of clinical trials.

The essence of the proposed invention is the use of a well-known drug sulfasalazine of the general formula I as an inhibitor of the formation of AGEs, which has a higher antiglycation activity compared to the prototype (aminoguanidine):

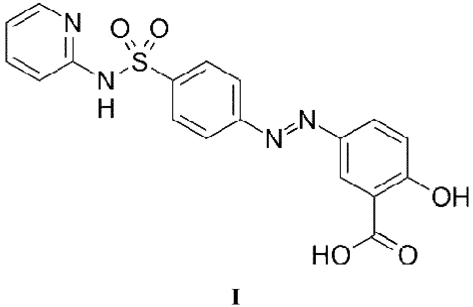

### Example of the claimed technical solution implementation

### Example: Determination of antiglycation activity

The glycation reaction is reproduced according to the method of A. Jedsadayanmata ([14] A. Jedsadayanmata, Naresuan University Journal, 2005, 13(2): 35-41). The reaction mixture contains solutions of bovine serum albumin (1 mg/ml) and glucose (500 mM) in phosphate buffer (pH = 7.4). To prevent bacterial growth, sodium azide in a final concentration of 0.02% is added to the buffer solution. All substances are dissolved in dimethyl sulfoxide (DMSO). 30 µ1 of sulfasalazine solution in various concentrations is added to the experimental samples; DMSO is added to the control samples in the same volume. All experimental samples are incubated for 24 hours at 60°C. At the end of the incubation period, specific fluorescence of glycated bovine serum albumin (BSA) is determined using an F-7000 spectrofluorimeter (Hitachi, Japan) at an excitation wavelength of 370 nm and an emission wavelength of 440 nm. As a comparison substance, a known glycation inhibitor aminoguanidine is used (Table 1) ([15] P.J. Thornalley, Archives of Biochemistry and Biophysics, 2003, 419: 31-40).

Statistical processing of the results is carried out using the non-parametric Mann-Whitney test, the Microsoft Excel 2007 table editor and the GraphPad Prism 5.0 software. The calculation of the IC₅₀ indicator is carried out by the method of regression analysis (Table 2).

**Table 1**

| The effect of sulfasalazine and aminoguanidine on BSA glycation at various concentrations | | | | | |
|---|---|---|---|---|---|
| **Drug concentration (mol) Drug name** | 10⁻³ | 3∗10⁻⁴ | 10⁻⁴ | 3∗10⁻⁵ | 10⁻⁵ |
| - | The average concentration of AGEs in the sample at a given concentration of the drug (Δ%, M±m) | | | | |
| Sulfasalazine | 98.94±0.20* | 97.53±0.43* | 81.60±7.18* | 65.37±2.25* | 43.42±4.21* |
| Aminoguanidine | 58.08±0.72* | 50.25±0.72* | 38.75±1.52* | 19.14±2.24* | 3.53±2.31* |

| | | | | | |
|---|---|---|---|---|---|
| Note: * the data are reliable in relation to the positive control (Mann-Whitney test, p <0.05). | | | | | |

**Table 2**

| IC₅₀ values for BSA glycation | |
|---|---|
| Substance | IC₅₀, µM |
| Sulfasalazine **I** | **23.90** |
| Aminoguanidine | 765.00 |

The analysis of the data shown in Table 1 and Table 2 allows us to conclude that **sulfasalazine exhibits a high level of antiglycation activity** (Table 1), which allows us to determine the dependence of its effect on concentration and calculate the IC₅₀ index (Table 2).

Thus, the results obtained by the applicant demonstrate that the activity of sulfasalazine significantly exceeds that of aminoguanidine, which allows us to consider it as an effective inhibitor of the formation of glycation end-products. At the same time, unlike aminoguanidine, sulfasalazine is an approved drug that has proven its high effectiveness and safety during many years of trials and clinical use.

Based on the foregoing, we can conclude that the claimed technical solution allows to create new highly effective and safe drugs for the prevention and treatment of micro- and macrovascular complications of diabetes, atherosclerosis, neurodegenerative diseases, cataract, and age-related diseases, thereby improving the quality of life of patients.

The claimed technical solution meets the criterion of "novelty" imposed on inventions, as the prior art study has not detected any technical solutions with the claimed combination of distinctive features that achieve the claimed results.

The claimed technical solution meets the criterion of "inventive step" imposed on inventions, as it is not obvious to a specialist in this field of science and technology.

The claimed technical solution meets the criterion of "industrial applicability", as it can be implemented at any specialized enterprise using standard equipment, well-known domestic materials and technologies.

## Claims

1. The use of Sulfasalazine (international non-proprietary name; synonyms: Salazosulfapyridine, Azopyrine, Asulfidine, Salazopyridin, Salazopyrin, Salazosulfapyridine, Salicylazosulfapyridin, Salisulf, Sulfasalazyn, Sulphasalazine) of general formula I for a new purpose:
